# EUROPEAN PATENT SPECIFICATION

(11) **EP 2 717 802 B1**
(45) Date of publication and mention of the grant of the patent: **31.10.2018**
(21) Application number: 12800458.7
(22) Date of filing: 13.06.2012
(51) Int. Cl.: A61F 2/95

(54) **RECONSTRAINABLE STENT DELIVERY SYSTEM**
WIEDER EINZIEHBARES STENTABGABESYSTEM
SYSTÈME DE POSE D'ENDOPROTHÈSE POUVANT ÊTRE DE NOUVEAU CONTRAINTE

(30) Priority: 13.06.2011 US 201161496376 P; 12.06.2012 US 201213494567
(43) Date of publication of application: 16.04.2014
(73) Proprietor: Flexible Stenting Solutions, Inc., Eatontown, NJ 07724-3255 (US)
(72) Inventor: BEACH, Bradley, Belmar, NJ 07719 (US); BURPEE, Janet, Fair Haven, NJ 07704 (US); FILACHEK, Andrew, Beachwood, NJ 08722 (US); KALAVALAPALLY, Rajesh, Ocean, NJ 07712 (US); SHAH, Neel, Edison, NJ 08817 (US); JAEGER, Dana, Fair Haven, NJ 07704 (US)
(74) Representative: Prock, Thomas
(86) International application number: PCT/US2012/042185
(87) International publication number: WO 2012/174069

(56) References cited:
- EP-A1- 2 208 483
- WO-A1-2004/098692
- WO-A1-2009/124124
- WO-A1-2010/130443
- US-A1- 2005 256 562
- US-A1- 2008 125 849
- US-A1- 2009 036 966
- US-A1- 2010 094 394
- US-B1- 6 254 628
- US-B1- 7 942 924

## Description

### Background of the Invention

### 1. Field of the invention

This invention pertains to a delivery system for a self expanding stent. The delivery system allows for reconstraining the stent into the delivery catheter simultaneously allowing the stent to change lengths and rotate inside the delivery catheter if required. This invention also pertains to a delivery system for self expanding stent that foreshortens an appreciable amount, for example, more than about 10%.

### 2. Description of the Related Art

Most commercial self expanding stents are not designed to be recaptured (reconstrained) into the delivery system once the stent has started to expand into the target vessel, artery, duct or body lumen. It would be advantageous for a stent to be able to be recaptured after the stent has started to deploy in the event that the stent is placed in an incorrect or suboptimal location, the stent could be recaptured and redeployed or recaptured and withdrawn. A recapturable stent and delivery system would constitute a major safety advantage over non-recapturable stent and delivery systems.

Many conventional self expanding stents are designed to limit the stent foreshortening to an amount that is not appreciable. Stent foreshortening is a measure of change in length of the stent from the crimped or radial compressed state as when the stent is loaded on or in a delivery catheter to the expanded state. Percent foreshortening is typically defined as the change in stent length between the delivery catheter loaded condition (crimped) and the deployed diameter up to the maximum labeled diameter divided by the length of the stent in the delivery catheter loaded condition (crimped). Stents that foreshorten an appreciable amount are subject to more difficulties when being deployed in a body lumen or cavity, such as a vessel, artery, vein, or duct. The distal end of the stent has a tendency to move in a proximal direction as the stent is being deployed in the body lumen or cavity. Foreshortening may lead to a stent being placed in an incorrect or suboptimal location. Delivery systems that can compensate for stent foreshortening would have many advantages over delivery systems that do not.

A stent is a tubular structure that, in a radially compressed or crimped state, can be inserted into a confined space in a living body, such as a duct, an artery or other vessel. After insertion, the stent can be expanded radially at the target location. Stents are typically characterized as balloon-expanding (BX) or self-expanding (SX). A balloon-expanding stent requires a balloon, which is usually part of a delivery system, to expand the stent from within and to dilate the vessel. A self expanding stent is designed, through choice of material, geometry, or manufacturing techniques, to expand from the crimped state to an expanded state once it is released into the intended vessel. In certain situations higher forces than the expanding force of the self expanding stent are required to dilate a diseased vessel. In this case, a balloon or similar device might be employed to aid the expansion of a self expanding stent.

Stents are typically used in the treatment of vascular and non-vascular diseases. For instance, a crimped stent may be inserted into a clogged artery and then expanded to restore blood flow in the artery. Prior to release, the stent would typically be retained in its crimped state within a catheter and the like. Upon completion of the procedure, the stent is left inside the patient's artery in its expanded state. The health, and sometimes the life, of the patient depend upon the stent's ability to remain in its expanded state.

Many conventional stents are flexible in their crimped state in order to facilitate the delivery of the stent, for example within an artery. Few are flexible after being deployed and expanded. Yet, after deployment, in certain applications, a stent may be subjected to substantial flexing or bending, axial compressions and repeated displacements at points along its length, for example, when stenting the superficial femoral artery. This can produce severe strain and fatigue, resulting in failure of the stent.

A similar problem exists with respect to stent-like structures. An example would be a stent-like structure used with other components in a catheter-based valve delivery system. Such a stent-like structure holds a valve which is placed in a vessel.

Document EP2208483 A1 discloses a stent delivery system which allows for reconstraining a stent after partial deployment.

### Summary of the Invention

The present invention as defined by claim 1 comprises a catheter delivery system for self-expanding stents. The reconstrainable stent delivery system of the present invention comprises a proximal end and distal end. An outer member is typically a shaft of a catheter or outer sheath of the catheter. A slider is positioned to interface at the proximal end of a crimped stent. The slider can rotate about and move longitudinally along one of an inner shaft or tube, such as the guide wire tube, such that the proximal end of the stent can move distally as the stent deploys. A pusher can be used on the guide wire tube such that the guide wire tube, pusher, and stent move proximally relative to the outer sheath and re-constrain the stent in the outer sheath. Furthermore, the pusher and guide wire tube could move distally as the outer sheath retracts proximally for stent deployment to accommodate foreshortening.

The delivery system can also include a spring element in the catheter delivery system to be incorporated or interfaced to the pusher and the spring element reacts the axial load at the proximal end of the stent during stent deployment. The spring element can bias the axial movement of the stent inside the delivery catheter to move distally as the stent is deployed. This biased movement is beneficial for stents that foreshorten an appreciable amount as the biased movement reduce the amount of movement at the distal end of the stent during stent deployment. The delivery system can include a housing as a means to grip a spring element. The spring element maintains the guide wire tube in tension during reconstraining of the stent.

In accordance with the invention the slider includes interlocking features that mate and lock with matching interlocking features on the stent. A gripper is coaxial to an outer sheath on the stent delivery system near the handle such that the gripper is always outside the body. During reconstraining, it may be beneficial for the user (physician) to grip the outer sheath and the pusher, thereby holding the pusher substantially stationary and moving the outer sheath distally to reconstrain the stent into the outer sheath. The gripper can be free to move axially along the outer sheath, and grip the outer sheath when the user applies pressure to the gripper or otherwise engages the gripper to the outer sheath.

The catheter delivery system can be used to deploy stents in iliac, femoral, popliteal, carotid, neurovascular or coronary arteries, treating a variety of vascular disease states.

An exemplary stent for use with the present invention combines a helical strut member or band interconnected by coil elements. This structure provides a combination of attributes that are desirable in a stent, such as, for example, substantial flexibility, stability in supporting a vessel lumen, cell size and radial strength. However, the addition of the coil elements interconnecting the helical strut band complicates changing the diameter state of the stent. Typically a stent structure must be able to change the size of the diameter of the stent. For instance, a stent is usually delivered to a target lesion site in an artery while in a small diameter size state, then expanded to a larger diameter size state while inside the artery at the target lesion site. The structure of the stent of the present invention provides a predetermined geometric relationship between the helical strut band and interconnected coil elements in order to maintain connectivity at any diameter size state of the stent.

The stent is a self expanding stent made from superelastic nitinol. Stents of this type are manufactured to have a specific structure in the fully expanded or unconstrained state. Additionally, a stent of this type must be able to be radially compressed to a smaller diameter, which is sometimes referred to as the crimped diameter. Radially compressing a stent to a smaller diameter is sometimes referred to as crimping the stent. The difference in diameter of a self expanding stent between the fully expanded or unconstrained diameter and the crimped diameter can be large. It is not unusual for the fully expanded diameter to be 3 to 4 times larger than the crimped diameter. A self expanding stent is designed, through choice of material, geometry, and manufacturing techniques, to expand from the crimped diameter to an expanded diameter once it is released into the intended vessel.

The stent comprises a helical strut band helically wound about an axis of the strut. The helical strut band comprises a wave pattern of strut elements having a plurality of peaks on either side of the wave pattern. A plurality of coil elements are helically wound about an axis of the stent and progress in the same direction as the helical strut band. The coil elements are typically elongated where the length is much longer than the width. The coil elements interconnect at least some of the strut elements of a first winding to at least some of the strut elements of a second winding of the helical strut band at or near the peaks of the wave pattern. In the stent a geometric relationship triangle is constructed having a first side with a leg length L_{C} being the effective length of the coil element between the interconnected peaks of a first and second winding of the helical strut band, a second side with a leg length being the circumferential distance between the peak of the first winding and the peak of the second winding interconnected by the coil element divided by the sine of an angle Aₛ of the helical strut band from a longitudinal axis of the stent, a third side with a leg length being the longitudinal distance the helical strut band progresses in 1 circumference winding (Pl) minus the effective strut length L_{S}, a first angle of the first leg being 180 degrees minus the angle Aₛ, a second angle of the second leg being an angle A_{c} the coil element generally progresses around the axis of the stent measured from the longitudinal axis and a third angle of the third leg being the angle Aₛ minus the angle A_{c}, wherein a ratio of the first leg length L_{C} to a length L_{S} multiplied by the number of adjacent wave pattern of the strut elements forming the helical strut band, N_{S} is greater than or equal to about 1. This value is defined as the coil-strut ratio and numerically is represented by coil-strut ratio= Lc/Ls*Ns.

### Brief Description of the Drawings

The foregoing description, as well as further objects, features, and advantages of the present invention will be understood more completely from the following detailed description of presently preferred, but nonetheless illustrative embodiments in accordance with the present invention, with reference being had to the accompanying drawings, in which:
Fig. 1 is a schematic drawing of a stent delivery system in accordance with the present invention.
Fig. 2 is a detailed enlarged view of X-X section shown in Fig. 1 just prior to stent deployment.
Fig. 3 is a detailed enlarged view of X-X section shown in Fig. 1 just prior to stent recapturing.
Fig. 4 is a detailed enlarged view of X-X section shown in Fig. 1 in an alternate embodiment configuration.
Fig. 5 is a detailed enlarged view of X-X section shown in Fig. 1 in an alternate embodiment configuration
Fig. 6 is a view of Z-Z section shown in Fig. 5 in an alternate embodiment configuration.
Fig. 7 is a detailed enlarged view of X-X section shown in Fig. 1 just prior to the start of stent deployment.
Fig. 8 is a detailed enlarged view of X-X section shown in Fig. 1 during stent deployment.
Fig. 9 is a schematic drawing of an alternate embodiment of the stent delivery system in accordance with the present invention.
Fig. 10 is a plan view of a first embodiment of a stent which can be used in the stent delivery system in accordance with the present invention, the stent being shown in a partially expanded state.
Fig. 11 is a detailed enlarged view of portion A shown in Fig. 1.
Fig. 12 is a plan view of an alternate embodiment of the stent.
Fig. 13 is an enlarged detailed view of portion B shown in Fig. 3.
Fig. 14 is a plan view of an alternate embodiment of the stent.
Fig. 15 is a plan view of an alternate embodiment of the stent.
Fig. 16 is a plan view of an alternate embodiment of the stent.
Fig. 17 is a detailed enlarged view of portion C shown in Fig. 7.
Fig. 18 is a plan view of an alternate embodiment of the stent.
Fig. 19 is a schematic diagram of an alternate embodiment for a coil element of the stent.
Fig. 20 is a detailed enlarged view of portion D shown in Fig. 14.
Fig. 21 is a detailed enlarged view of X-X section shown in Fig. 1 with an alternate embodiment configuration
Fig. 22 is a schematic drawing of an alternate embodiment of the stent delivery system in accordance with the present invention
Fig. 23 is schematic drawing of the stent delivery system in accordance with the present invention, as shown in Fig. 22, where certain elements are shown in cross section and prior to reconstraining the stent.
Fig. 24 is schematic drawing of the stent delivery system in accordance with the present invention, as shown in Fig. 22, where certain elements are shown in cross section and during stent reconstraining.
Fig. 25 is a schematic drawing of an alternate embodiment of the stent delivery system in accordance with the present invention.
Fig. 26 is a schematic drawing of an alternate embodiment of the stent delivery system in accordance with the present invention.
Fig. 27 is a plan view of an embodiment of a stent and slider in accordance with the present invention, the stent being shown in a crimped state. In this view the stent and slider are interlocked.
Fig. 28 is a plan view of an embodiment of a stent and slider in accordance with the present invention, the stent being shown in a crimped state. In this view the stent and slider are not interlocked.
Fig. 29 is a schematic drawing of an alternate embodiment of the stent delivery system in accordance with the present invention.

### Detailed Description of the invention

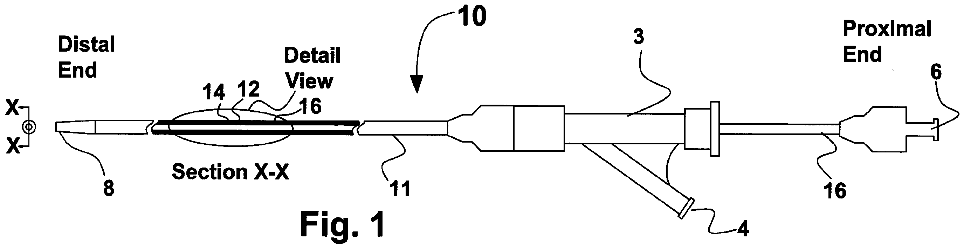

Self expanding stent delivery system 10 of the present invention is shown in Fig. 1. The outer tube which is also known as outer sheath 11, constrains stent 12 in a crimped or radially compressed state. The inner members can be comprised of multiple components including distal tip 8, guide wire tube 14 and pusher 16 to react the axial forces placed on the stent as outer sheath 11 is retracted to deploy stent 12. Pusher 16 can also act as a proximal stop. Other elements of self expanding stent delivery system 10 can include luer lock hub 6 attached to the proximal end of pusher 16 and handle 3 attached to outer sheath 11. Handle 3 incorporates luer port 4 such that the space between the inner members and outer sheath 11 can be flushed with a solution, such as a saline solution, to remove any entrapped air. Pusher 16 can be formed of a composite structure of multiple components, such as a stainless steel tube at the proximal end and a polymer tube inside outer sheath 11.

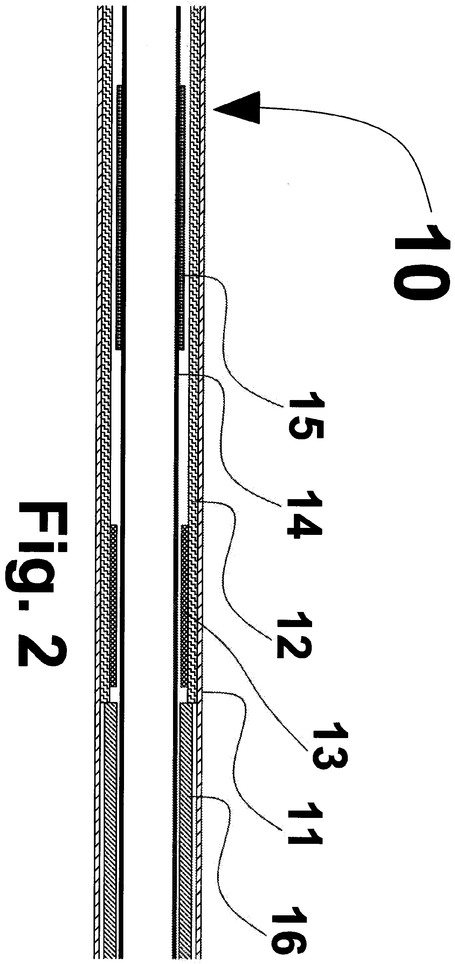

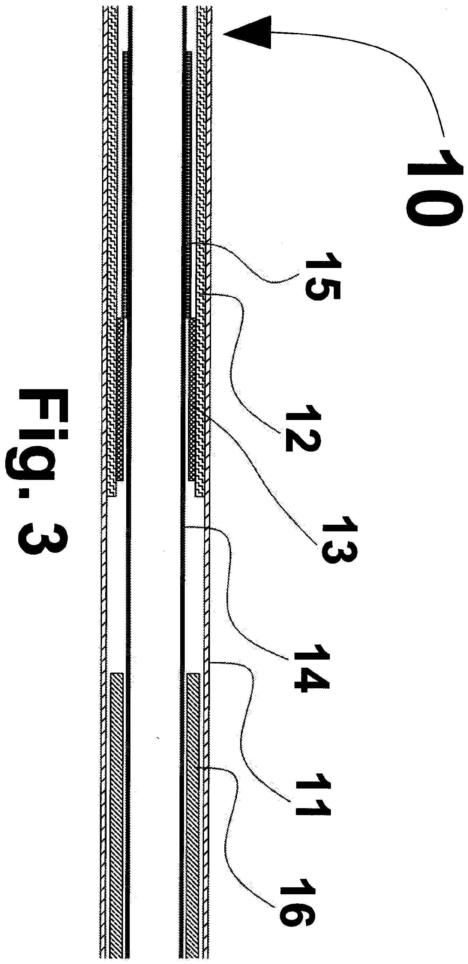

Stent delivery system 10 of the present invention, shown in the detail view of X-X section, Fig. 2, is comprised of outer sheath 11 in which stent 12 is constrained in a crimped, or radially compressed state. Stent delivery system 10 can be referred to as catheter delivery system as a delivery catheter. Slider 13 is positioned to interface with the inside diameter of crimped stent 12. Slider 13 is coaxial with guide wire tube 14 and slider 13 is free to rotate and slide relative to guide wire tube 14. Distal stop 15 is fixed to guide wire tube 14 at a position distal to slider 13. Pusher 16 is positioned proximal to stent 12 and slider 13 and reacts the axial forces transmitted to stent 12 as outer sheath 11 is retracted to deploy the stent and provides a proximal stop. Stent 12 and slider 13 are free to move, translate or rotate, within outer sheath 11 and relative to guide wire tube 14 as outer sheath 11 is retracted and stent 12 is deployed. This is advantageous when the stent design is such that stent 12 shortens in length and/or rotates as it expands from the crimped state to a larger diameter expanded state. The delivery system of the present invention allows the stent movement to occur inside outer sheath 11 instead of inside the body lumen. Before outer sheath 11 is fully retracted, thereby releasing stent 12, the stent can be recaptured by moving guide wire tube 14 and attached distal stop 15 proximally relative to stent 12 and slider 13 until distal stop 15 contacts slider 13, as shown in detail view of X-X section, Fig. 3. Because stent 12 and slider 13 are intimate contact with each other, outer sheath 11 can be moved distally relative to stent 12, slider 13, guide wire tube 14 and distal stop 15, there by recapturing stent 12 inside outer sheath 11. In this embodiment, pusher 16 is in contact with stent 12 as outer sheath 11 is retracted to deploy stent 12.

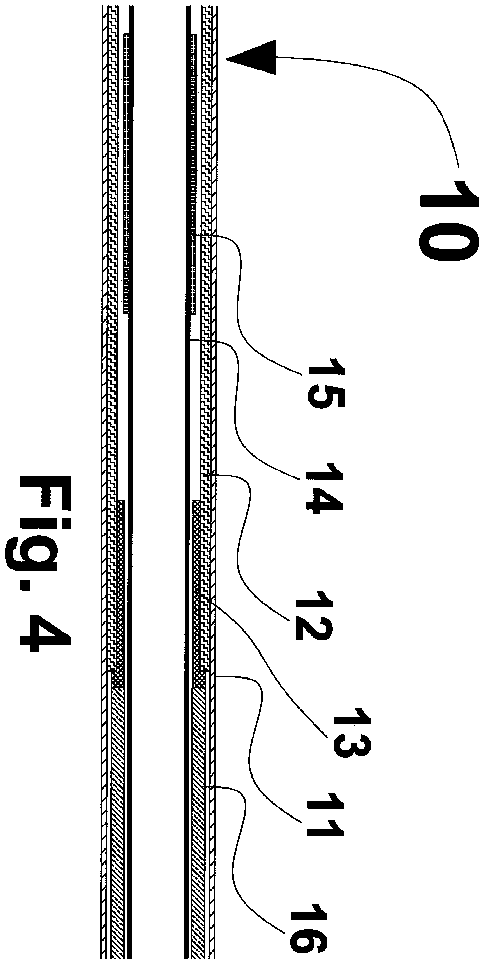

In an alternate embodiment, slider 13 is designed to interface with the inside diameter of stent 12 and contact pusher 16 as outer sheath 11 is retracted, as shown in Fig. 4. This embodiment reduces the axially load directly placed on stent 12 during stent deployment.

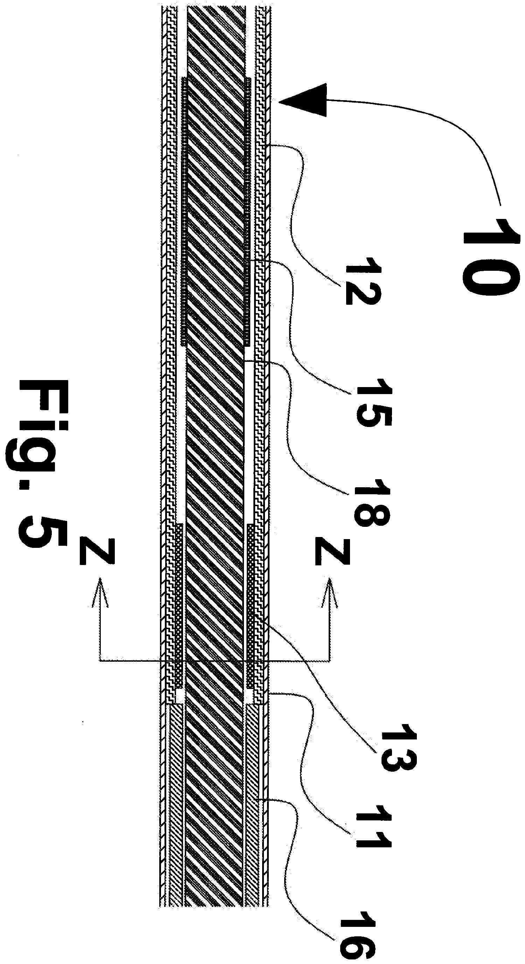

In the embodiment described above, slider 13 is coaxial with guide wire tube 14 and slider 13 is free to rotate and slide relative to guide wire tube 14. Guide wire tube 14 can be hollow, forming a lumen that runs the length of the stent delivery system to accommodate a guide wire which is often used to facilitate locating the stent delivery system in the target vessel, artery, duct or body lumen. Alternatively, guide wire tube 14 can be a non-hollow solid shaft 18 as shown in Fig. 5.

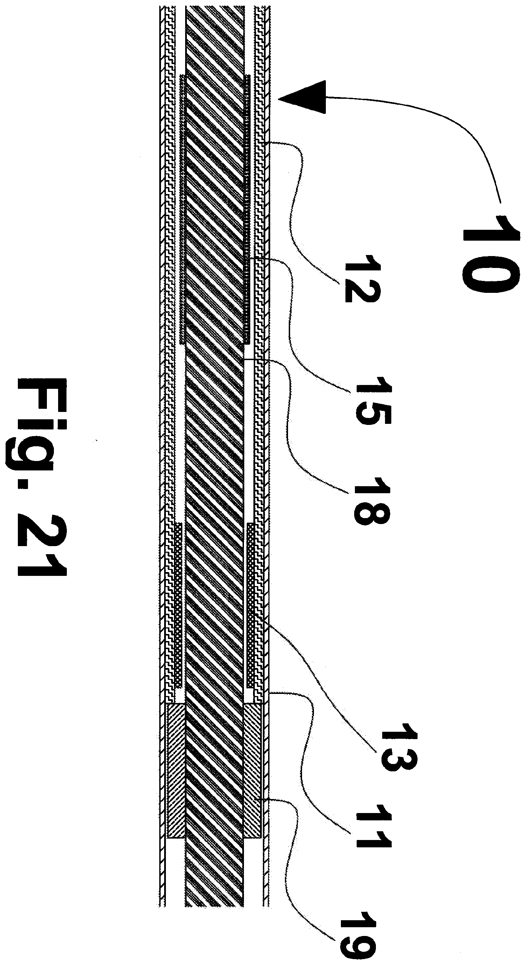

In an alternate embodiment, axial force at the proximal end of the stent is reacted by a proximal stop 19, attached to non-hollow shaft 18, such that proximal stop 19 and non-hollow shaft are a unitary member, as shown in Fig. 21. Proximal stop 19 and non-hollow shaft 18 could be made from different materials that are affixed together or made from the same material.

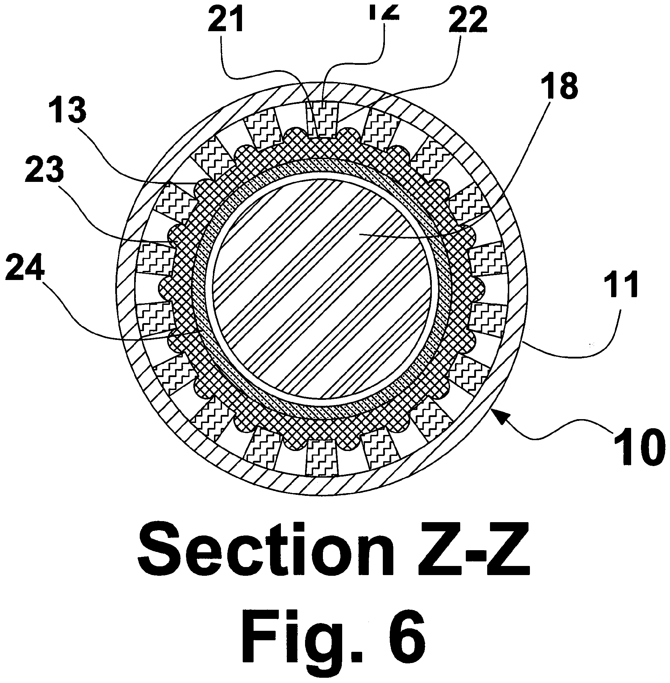

In an alternate embodiment shown in Z-Z section view, Fig. 6, slider 13 is formed of a structure where a portion of slider 13 is a polymer that is molded or formed to inside diameter 21 of stent 12 and/or sidewall 22 of stent 12. Slider 13 can be a composite or laminated structure comprising polymer portion 23 interfacing with stent 12 and rigid portion 24 near the inside diameter of slider 13.

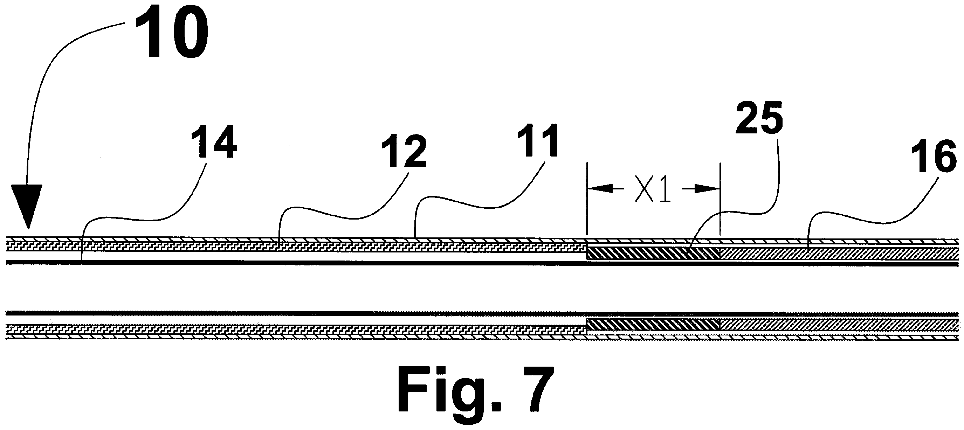

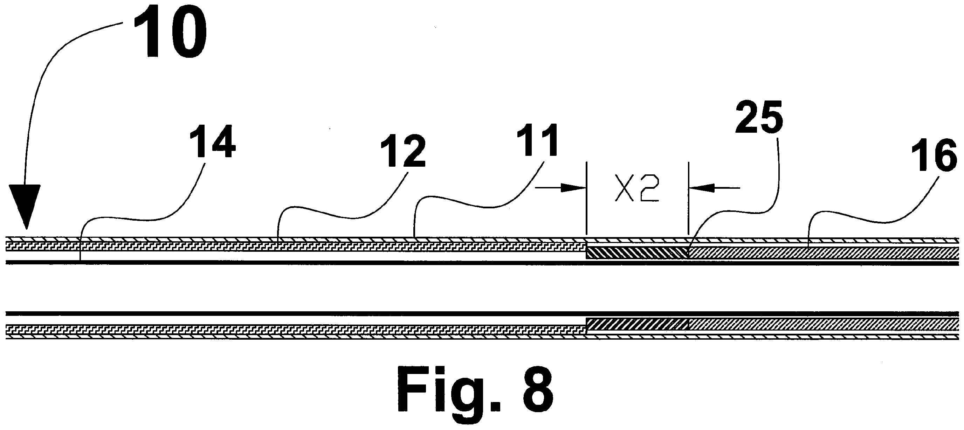

In another embodiment as shown in detail view of X-X section, Fig. 7 and Fig. 8, spring element 25 is incorporated into pusher 16 such that spring element 25 is compressed as the axial force at the proximal end of stent 12 increases until outer sheath 11 starts to move in a proximal direction relative to stent 12. As stent 12 deploys, spring element 25 continues to react the axial load at the proximal end of stent 12 and simultaneously pushes the proximal end of stent 12 distally as stent 12 foreshortens coming out of outer sheath 11. Fig. 7 shows spring element 25 in an uncompressed state prior to the start of stent 12 deployment where stent 12 is not under an axial load. Fig. 8 shows spring element 25 in a compressed state after the start of deployment where stent 12 is under an axial load, where X2<X1. As stent 12 expands out of outer sheath 11, the axial load on stent 12 will typically decrease from a peak load near the beginning of the deployment. As the axial load decreases, the spring force will push the proximal end of stent 12 forward to bias any movement of stent 12 due to foreshortening occurring at the proximal end of stent 12, such that the proximal end of stent 12 moves distally instead of the distal end of stent 12 moving proximally.

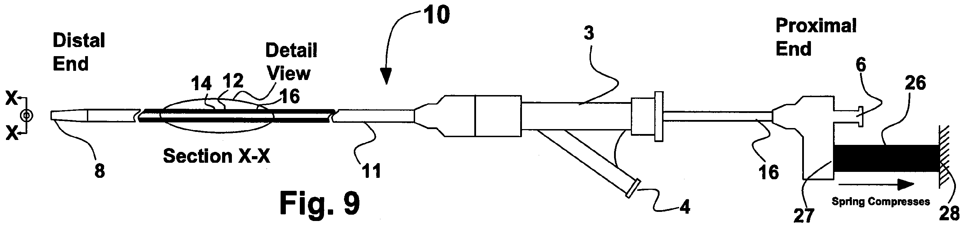

In an alternate embodiment, spring element 26 can be incorporated at the proximal end of stent delivery system 10, where distal end 27 of spring element 26 effectively interfaces with pusher 16, and proximal end 28 of spring element 26 is fixed, as shown in Fig. 9. Pusher 16 compresses spring element 26 as the axial force at the proximal end of stent 12 increases until outer sheath 11 starts to move in a proximal direction relative to stent 12. As stent 12 deploys, spring element 26 moves pusher 16 proximally as stent 12 foreshortens coming out of outer sheath 11.

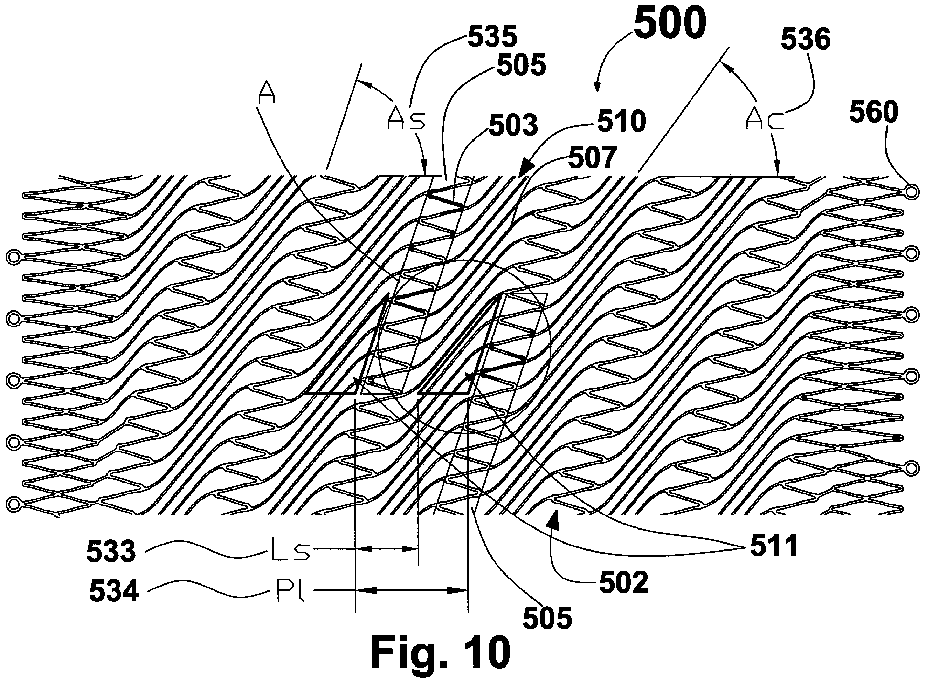

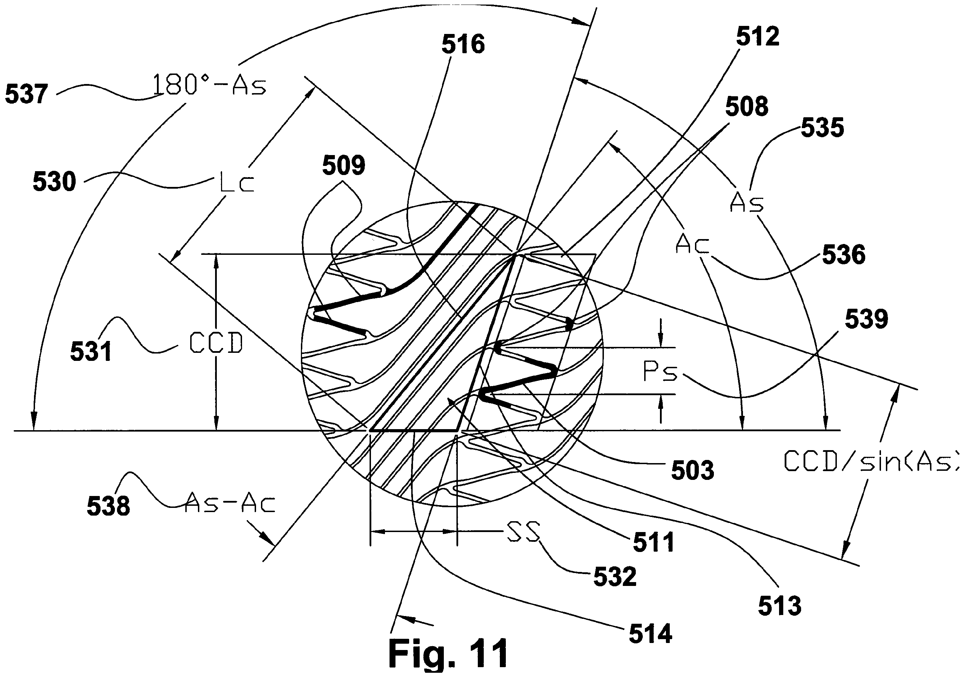

Fig. 10 with detail shown in Fig. 11 illustrates stent 500 which can be used in stent delivery system 10. Fig 10 is a plan view of a first embodiment of stent 500 shown in a partially expanded state. As used herein, the term "plan view" will be understood to describe an unwrapped plan view. This could be thought of as slicing open a tubular stent along a line parallel to its axis and laying it out flat. It should therefore be appreciated that, in the actual stent, the top edge of Fig. 10 will be joined to the lower edge. Stent 500 is comprised of helical strut band 502 interconnected by coil elements 507. Side-by-side coil elements 507 form coil band 510. Coil band 510 is formed as a double helix with helical strut band 502 and progresses from one end of the stent to the other. Helical strut band 502 comprises a wave pattern of strut elements 503 that have peaks 508 on either side of the wave pattern and legs 509 between peaks 508. Coil elements 507 interconnect strut elements 503 of helical strut band 502 through or near peaks 508. NSC portion 505 of helical strut band 502 is defined by the number of strut elements 503 (NSC) of helical strut band 502 between coil element 507 as helical strut band 502 progresses around stent 500. The number of strut elements 503 (NSC) in NSC portion 505 of helical strut band 502 is more than the number of strut elements 503 (N) in one circumference winding of helical strut band 502. The number of strut elements 503 (NSC) in NSC portion 505 is constant.

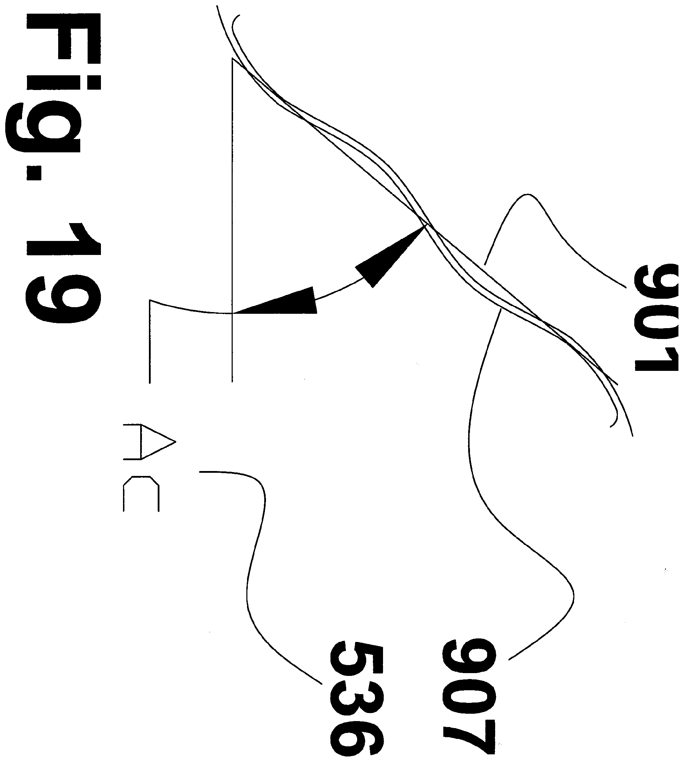

In this embodiment, stent 500 has N=12.728 helical strut elements 503 in one circumference winding of helical strut band 502 and has NSC=16.5 helical strut elements 503 in NSC portion 505. The number of helical strut elements in one circumference winding of helical strut band 502 has NSC greater than N+1. CCDn portion 512 of NSC portion 505 of helical strut band 502 is defined by the number of strut elements 503 (CCDn) equal to NSC minus N. The number of strut elements 503 (CCDn) in CCDn portion 512 and the number of strut elements 503 (N) in one circumference winding of helical strut band 502 does not need to be constant at different diameter size states of stent 500. Stent 500 has CCDn=3.772 helical strut elements 503 in CCDn portion 512. Because this connectivity needs to be maintained at any diameter size state a geometric relationship between the helical strut band 502 and coil element 507 can be described by geometric relationship triangle 511. Geometric relationship triangle 511 has a first side 516 with a leg length equal to the effective length (Lc) 530 of coil element 507, a second side 513 with a leg length equal to circumferential coil distance (CCD) 531 of CCDn portion 512 of helical strut band 502 divided by the sine of an angle Aₛ 535 of helical strut band 502 from the longitudinal axis of stent 500, a third side 514 with a leg length (SS) 532 equal to the longitudinal distance (Pl) 534 helical strut band 502 progresses in 1 circumference winding minus the effective strut length L_{S} 533, a first angle 537 of first side 516 is equal to 180 degrees minus angle Aₛ 535, a second angle 536 of second side 513 is equal to the angle A_{c} 536 of coil element 507 from the longitudinal axis of stent 500 and a third angle 538 of third side 514 equal to angle Aₛ 535 minus angle A_{c} 536. If the circumferential strut distance (Pₛ) 539 of helical strut element 503 is the same for all helical strut elements 503 in CCDn portion 512, circumferential coil distance CCD 531 is equal to the number of helical strut elements 503 in the CCDn portion 512 multiplied by the circumferential strut distance (Pₛ) 539. The distances in any figure that shows a flat pattern view of a stent represent distances on the surface of the stent, for example vertical distances are circumferential distances and angled distances are helical distances. First side 516 of geometric relationship triangle 511 is drawn parallel to the linear portion of coil element 507 such that the coil angle Ac 536 is equal to the angle of the linear portion of coil element 507. If coil element 507 does not have a substantially linear portion, but progresses about the stent in a helical manner, an equivalent coil angle 536 could be used to construct the geometric relationship triangle 511. For instance if coil element 507 is a wavy coil element 907, as shown in figure 19, line 901 could be drawn fitted through the curves of the wavy coil element 907 and line 901 can be used to define coil angle 536.

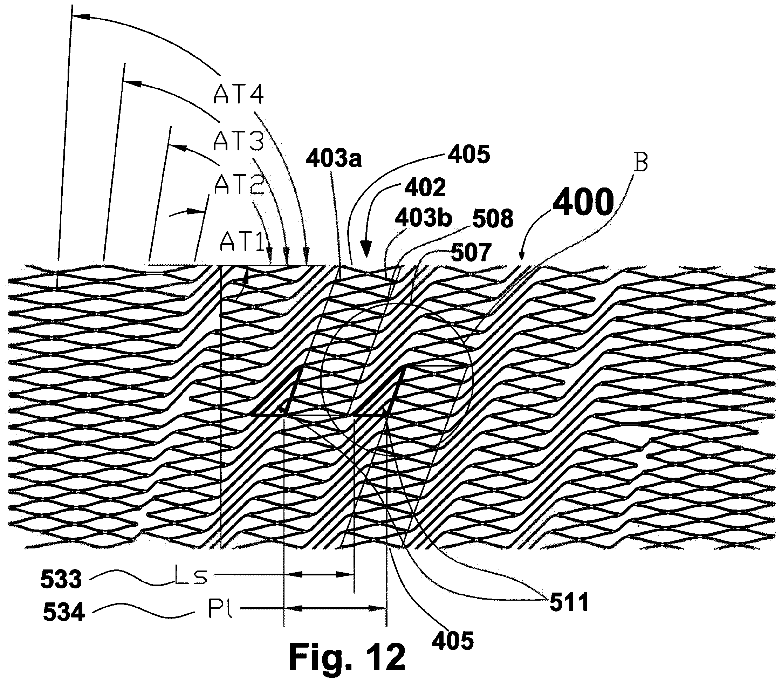

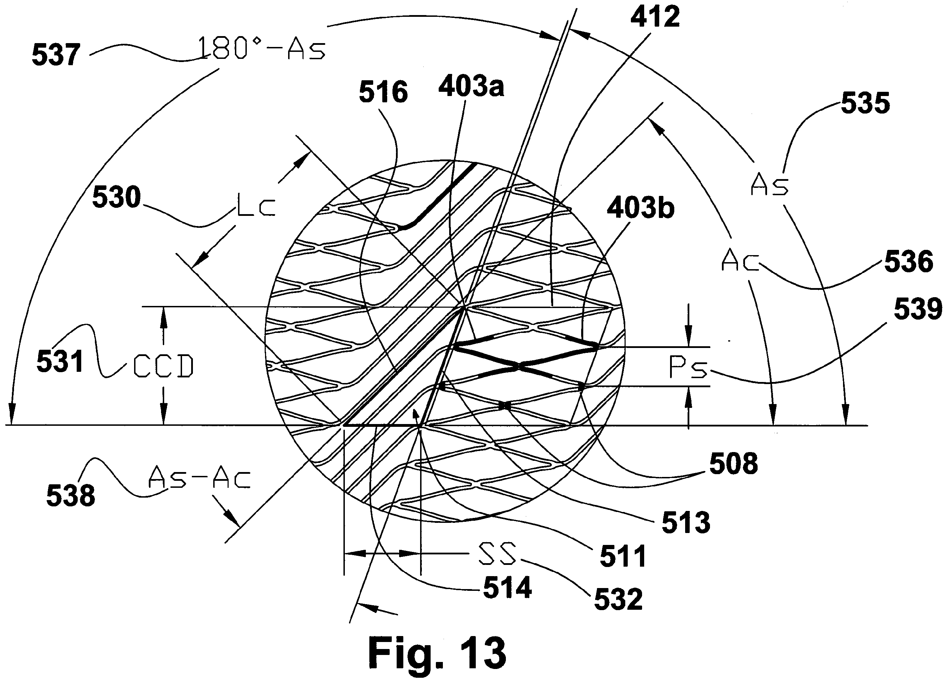

Stent 400 shown in Figs. 12 and 13 is similar to stent 500 in that it is comprised of helical strut band 402 interconnected by coil elements 507. Stent 400 is different in that helical strut band 402 is comprised of two adjacent wave patterns of strut elements 403a and 403b that have peaks 508 on either side of the wave pattern. Strut element 403a being connected to strut element 403b. Similar to helical strut band 502, helical strut band 402 also has a NSC portion 405 and a CCDn portion 412. Helical strut band 402 can be defined as having a number Ns of wave patterns of strut elements equal to 2. Helical strut band 502 can be defined as having a number Ns of wave patterns of strut elements equal to 1. In an alternate embodiment, the stent can have a helical strut band with a number Ns of wave patterns of strut elements equal to 3, which would be a triple strut band. In an alternate embodiment, the stent could have a helical strut band with a number Ns of wave patterns of strut elements equal to any integer. Stents with helical strut bands having a number Ns of wave patterns of strut elements equal to or greater than 2 provide an advantage in that the helical strut band would form a closed cell structure with smaller cell size which is desired when there is additional risk of embolism. Stents with smaller cell sizes tend to trap plaque or other potential embolic debris better than stents with larger cell sizes.

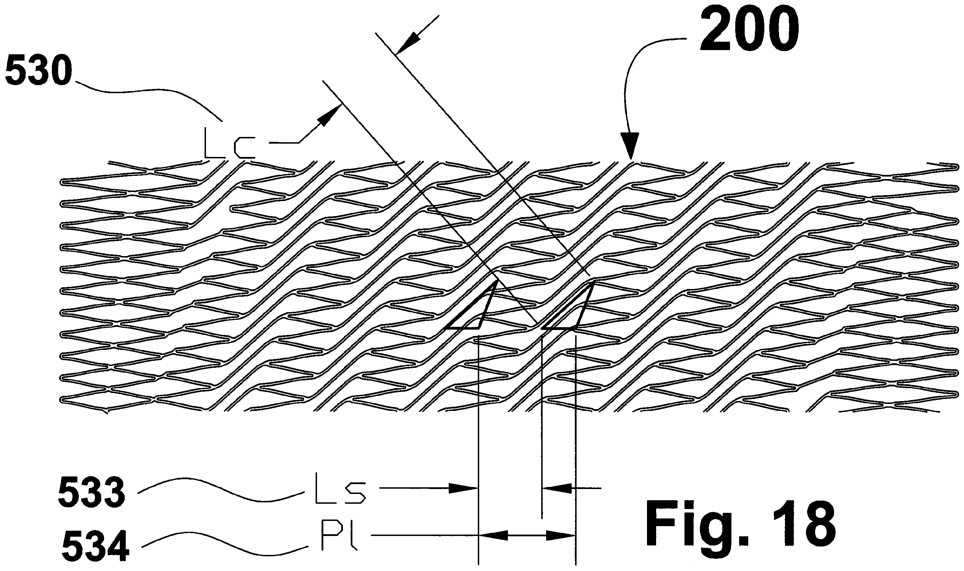

Stent structures described provides the combination of attributes desirable in a stent when the coil-strut ratio, ratio of Lc to Ls multiplied by the number of wave patterns of strut elements Ns in the helical strut band (Lc multiplied by Ns divided by Ls), is greater than or equal to 1. For example the coil-strut ratio for stent 500 is 2.06 and for stent 400 is 2.02. Stent 200 shown in Fig. 18 has a similar structure to stent 500. The coil-strut ratio for stent 200 is about 1.11.

In order for the stent to be crimped to a smaller diameter, the geometry of the structure undergoes several changes. Because of the helical nature of the helical strut band, strut angle Aₛ must get smaller as the stent diameter decreases. Because of the interconnectivity between a first winding of the helical strut band and a second winding of the helical strut band created by the coil element, the angle of the element A_{c} must also get smaller, or become shallower, to accommodate the smaller strut angle Aₛ. If the angle of coil element A_{c} can not become shallower or is difficult to become shallower as the stent crimps and stent angle Aₛ gets smaller, the coil elements will tend to interfere with each other and prohibit crimping or require more force to crimp. The changing of the angle of the coil element during crimping is facilitated if the coil-strut ratio is greater than 1. Coil-strut ratios less than 1 tend to stiffen the coil element such that more force is required to bend the coil element to a shallower angle during the crimping process, which is not desirable.

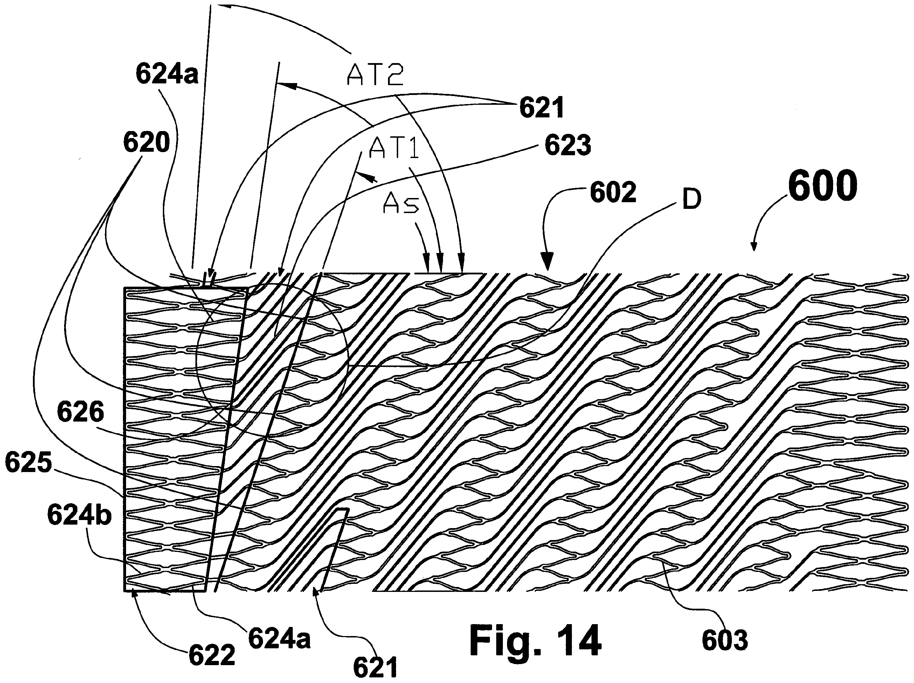

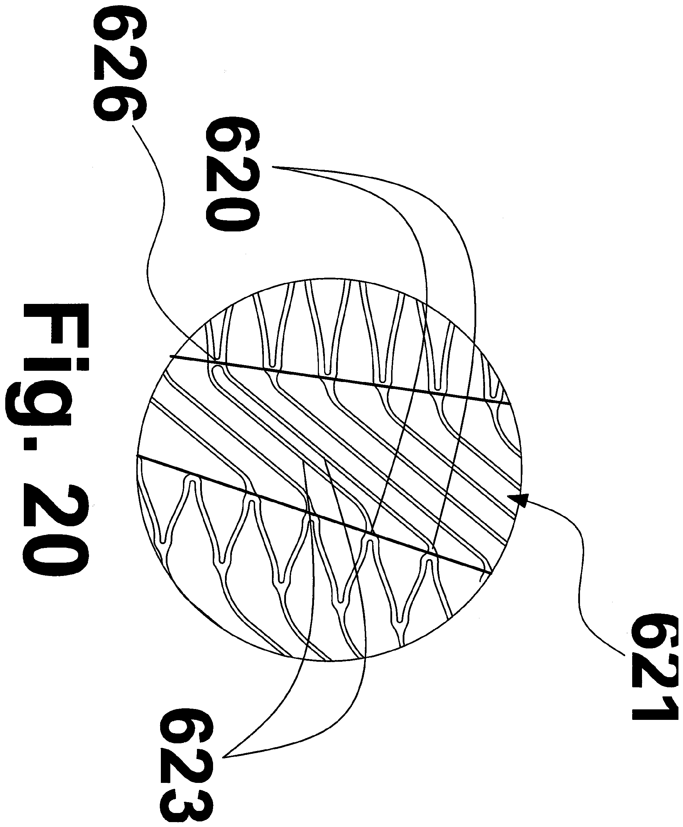

Helical strut band 602 of stent 600, shown in Fig. 14, transitions to and continues as an end strut portion 622 where the angle of the winding AT1 of the wave pattern of strut elements 624a forming end strut portion 622 is larger than the angle of the helical strut band Aₛ. End strut portion 622 includes a second winding of the wave pattern of strut elements 624b where the angle AT2 of the second winding is larger than the angle of the first winding AT1. Strut elements 603 of helical strut band 602 are interconnected to strut elements 624a of the first winding of end strut portion 622 by a series of transitional coil elements 623 that define transition coil portion 621. All strut elements 624a of the first winding of end portion 622 are connected by coil elements 623 to the helical strut band 602. Peaks 620 of helical strut band 602 are not connected to end strut portion 622. Transitional coil portion 621 allows end strut portion 622 to have a substantially flat end 625. Helical strut band 402 of stent 400 transitions to and continues as an end portion where the angle of the first winding AT1 of the wave pattern of strut elements forming of the end portion is larger than the angle of the helical strut band As. The angle of the second winding AT2 is larger than AT1, and the angle of subsequent windings of the end portion are also increasing (i.e. AT1<AT2<AT3<AT4). As shown in Fig. 20, stent 600 includes one peak 626 of end strut portion 622 connected to two peaks 620 of helical strut band 602 by transitional coil elements 623.

The accompanying definitions are described below.
- (N) - Number of helical strut elements in one circumference winding of the helical strut member.
- (Aₛ) - Angle of helical strut band winding measured from the longitudinal axis of the stent.
- (A_{c}) - Effective angle of coil element measured from the longitudinal axis of the stent.
- (Pl) - Longitudinal distance (pitch) the strut member progresses in 1 circumference winding. Equal to the circumference of the stent divided by the arctangent of Aₛ.
- (Pₛ) - Circumferential distance (pitch) between strut legs of a helical strut element of the helical strut band. Assuming the circumferential strut pitch is equal for all strut elements of the helical strut band, the circumferential strut pitch is equal to the circumference of the stent divided by N.
- (NSC) - Number of strut elements of the strut band between a helical element as the strut member progresses
- (CCDn) - Number of strut elements of the strut band between interconnected strut elements, equal to NSC minus N
- (CCD) - Circumferential Coil Distance is the circumferential distance between interconnected strut elements, equal to the CCDn times the Pₛ if the Ps is equal for all strut elements in the CCDn portion.
- (Lc) - Effective length of the helical element as defined by the geometric relationship triangle described in table 1.
- (SS) -Strut Separation as defined in the geometric relationship triangle described in table 1.
- (Ls) - Effective Strut Length. Equal to Pl minus SS.
- (Ns) - Number of adjacent wave patterns of the strut elements forming the helical strut band.
- Coil-Strut ratio - Ratio of L_{C} to a length L_{S} multiplied by the number of adjacent wave pattern of the strut elements forming the helical strut band, N_{S}. Numerically equal to Ns*Lc/Ls.
- Strut length-Strut Separation ratio - Ratio of the effective strut length (Ls) to the Strut Separation (SS), numerically equal to Ls/SS.

| Table 1 | | |
|---|---|---|
| | Leg Length | Angle |
| Side 1 | Lc | 180° minus Aₛ |
| Side 2 | CCD divided by sin(Aₛ) | A_{c} |
| Side 3 | SS | Aₛ minus A_{c} |

In one embodiment, the difference between the strut angle, Aₛ, and coil angle, A_{c}, is more than about 20 degrees. Because of the necessity of the coil angle to become shallower as the stent is crimped, if the coil angle and the strut angle in the expanded state are too close to each other there is increased difficulty in crimping the stent.

For the stent of the present invention the Strut length - Strut Separation ratio is a measure of the relative angle of the strut angle and coil angle. Stents with Strut length - Strut Separation ratios less than about 2.5 have improved crimping behavior. Stent attributes can further be improved if the angle of the strut member is between 55 degrees and 80 degrees and the coil angle is between 45 degrees and 60 degrees in the expanded state. Additionally, steeper coil angles A_{c} in the expanded state make crimping the stent of the present invention more difficult. Coil angles of less than 60 degrees in the expanded state facilitate crimping the stent.

For the stent in addition to the coil angle changing during crimping, the helical strut band rotates about the longitudinal axis of the stent to accommodate the connectivity between subsequent windings of helical strut bands during crimping resulting in more windings of the helical strut band along the length of the stent when the stent is crimped. In one embodiment, the longitudinal pitch of the helical strut band (Pl) is approximately the same in both the expanded state and crimped state. Considering that an increase of helical strut band windings along the length of the stent when the stent is crimped contributes to stent foreshortening it is advantageous for the stent of the present invention to have an approximated increase in the amount of helical strut band windings of less than about 30% when crimped, preferably less than about 26%. A 26% increase in helical strut band winding corresponds to about 20% foreshortening which is considered the maximum clinically useful amount of foreshortening (Serruys, Patrick, W., and Kutryk, Michael, J. B., Eds., Handbook of CoronaryStents, Second Edition, Martin Dunitz Ltd., London, 1998.).

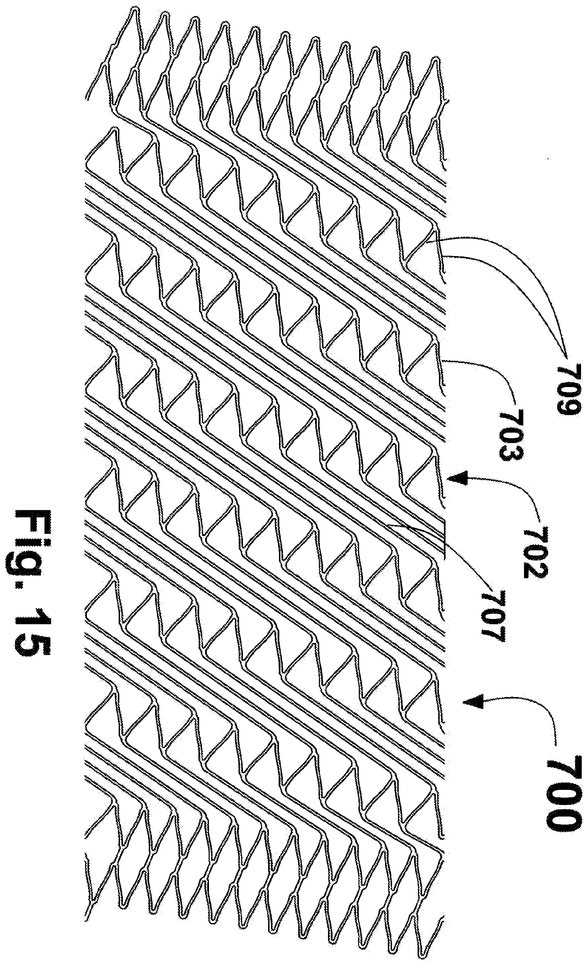

Fig. 15 is a plan view of another embodiment of stent 700. Helical strut band 702 progresses helically from one end of stent 700 to the other. Each strut element 703 is connected to a strut in a subsequent winding of helical strut band 702 by coil element 707. Strut element 703 includes leg portions 709. Each of leg portions 709 has an equal length.

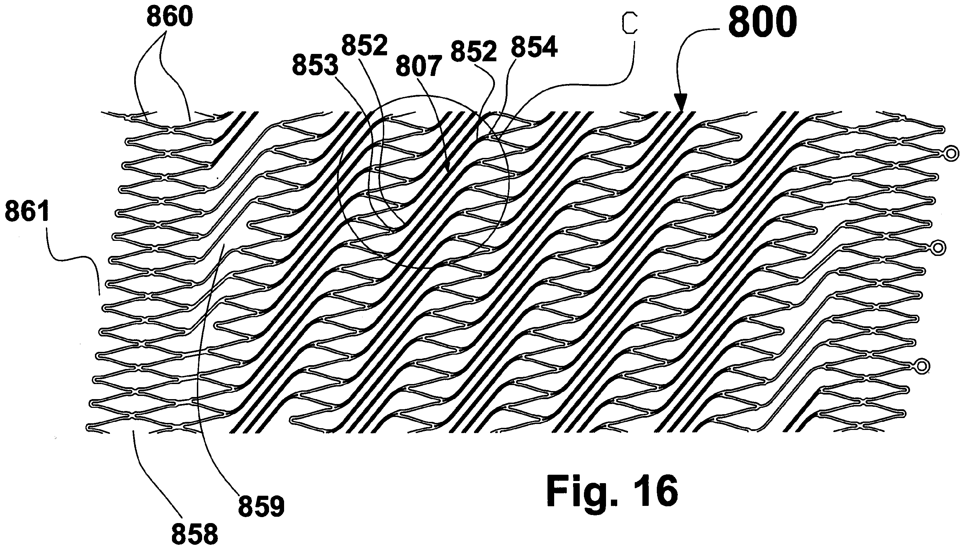

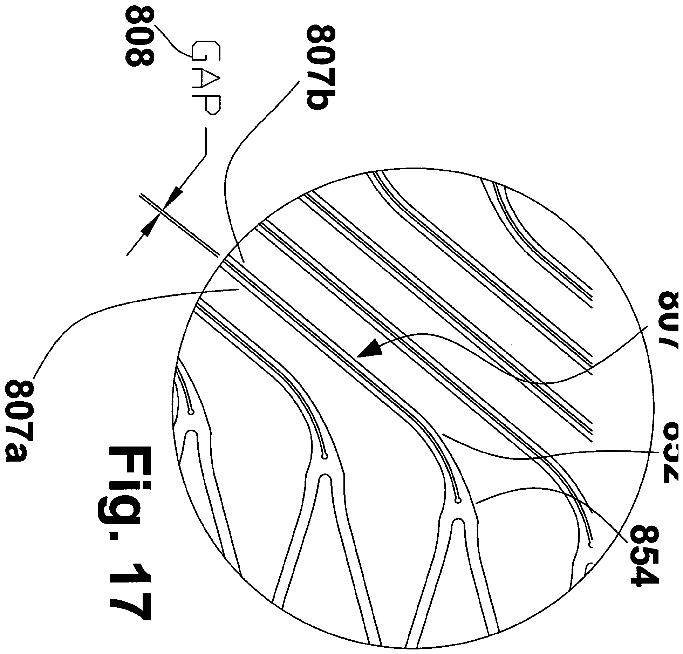

Fig. 16, with detail shown in Fig. 17, is a plan view of another embodiment of stent 800. In this embodiment, coil element 807 includes curved transition portion 852 at ends 853 and 854. Curved transition portion 852 connects to strut element 803.

Stent 800 includes transitional helical portions 859 and end strut portions 858 at either end 861 of stent 800. End strut portions 858 are formed of a pair of connected strut windings 860. Coil element 807 is comprised of two coil portions 807a and 807b which are separated by gap 808, as shown in Fig. 17. Gap 808 can have a size equal to zero where coil portions 807a and 807b are touching. Gap 808 terminates near ends 853 and 854. Gap 808 can terminate anywhere along the length of coil 807 or at multiple points along coil 807 such that the gap would have interruptions along coil 807.

Stents 400, 500, 600, 700 and 800 are made from a common material for self expanding stents, such as Nitinol nickel-titanium alloy (Ni/Ti), as is well known in the art.

In an alternate embodiment, stent 12 can be a stent as described in U.S. Patent No. 7,556,644.

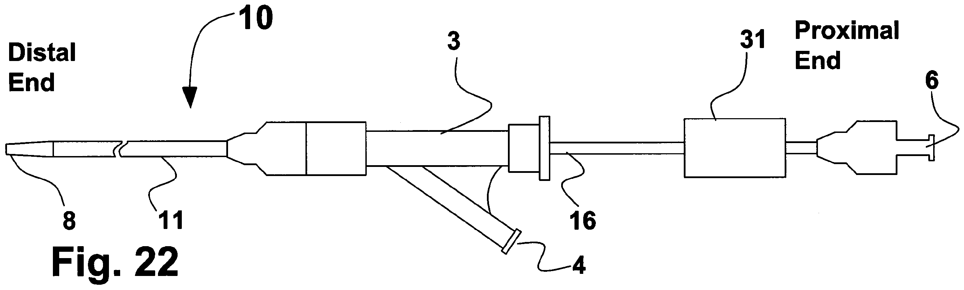

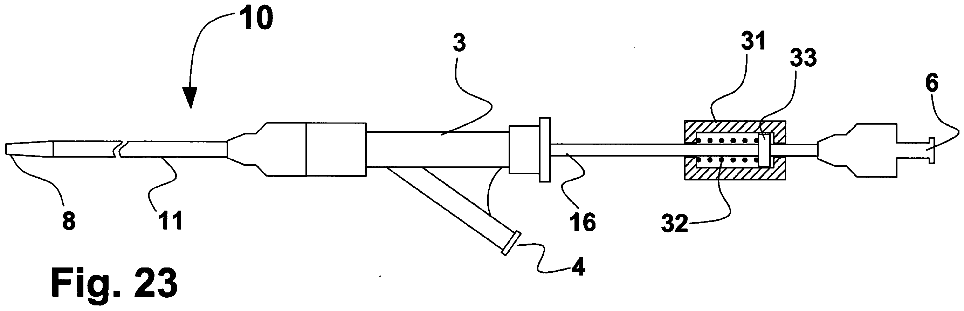

In an alternate embodiment as shown in Fig. 22, housing 31 is assembled to pusher 16 and is an interface/grip for the user during the recapturing/reconstraining of the stent after partial deployment. Pusher 16 is additionally coupled to guide wire tube 14 (not shown) such that as pusher 16 is moved guide wire tube 14 also moves. When the user chooses to reconstrain the stent after partial deployment, the user grips housing 31 and simultaneously moves outer sheath 11 and handle 3 in the proximal direction. Housing 31 contains compression spring element 32 and pusher stop 33, as shown in Fig. 23. Pusher stop 33 is attached to pusher 16.

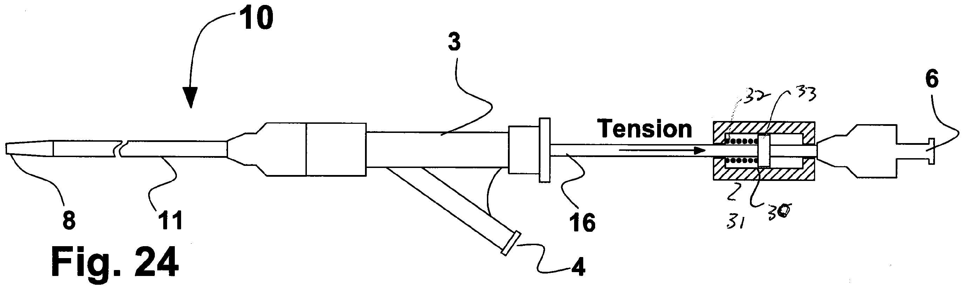

Fig. 23 shows system 10 prior to reconstraining the stent. Compression spring element 32 is in a relaxed or nearly relaxed state. As the stent is reconstrained, pusher stop 33 compresses compression spring element 32 against inner surface 30 of housing 31 such that guide wire tube 14 (not shown) is maintained under tension during at least some of the stent reconstraining, as shown in Fig. 24. This is advantageous when the stent has appreciable foreshortening. When stent 12 is recaptured compression spring element 32 will keep distal stop 15 (not shown) in contact with slider 13 (not shown).

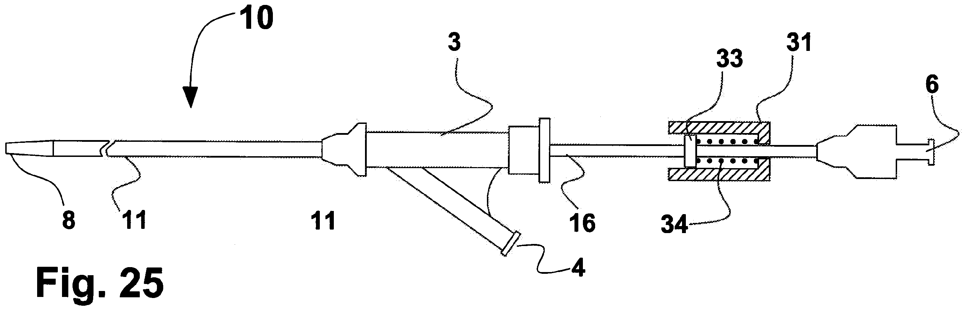

An alternate embodiment is shown in Fig. 25, wherein the spring element is tension spring element 34. Tension spring element 34 is coupled to inner surface 30 of housing 31 and pusher stop 33. This embodiment also maintains guide wire tube 14 under tension during at least some of the stent reconstraining. In these embodiments, housing 31 is used as means for the user to grip the spring elements. In an alternate embodiment, a spring element could be coupled directly to slider 13 to maintain direct tension on the stent during some of the stent reconstraining in accordance with the teachings of the present invention.

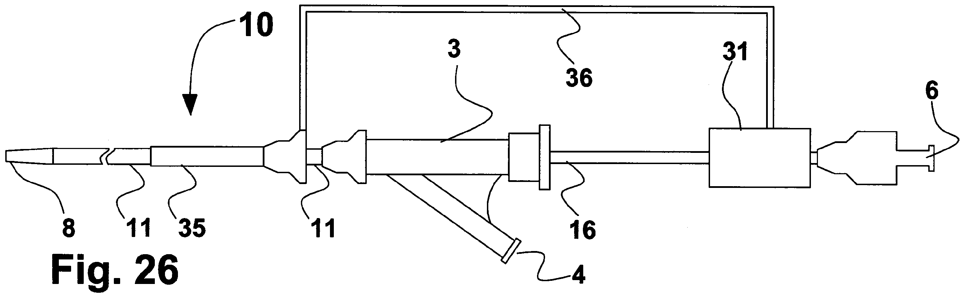

Fig. 26 shows an alternate embodiment having secondary outer sheath 35 which is outside and co-axial with outer sheath 11. Secondary outer sheath 35 is coupled to housing 31 by coupling member 36. Housing 31 and secondary outer sheath 35 move together or remain stationary together. In an alternate embodiment, secondary outer sheath 35 and housing 31 are not coupled to one another.

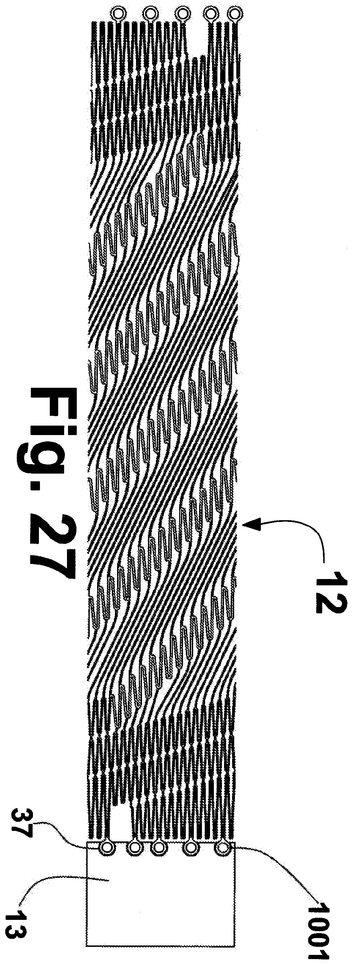

As shown in Fig 27, slider 13 according to the invention includes interlocking features 37 that mate and lock with matching interlocking features 1001 on stent 12. Interlocking features 1001 on stent 12 can be male or female, provided that interlocking features 37 on slider 13 are the opposite (e.g. male on stent and female on slider). Fig. 27 shows an example of male interlocking feature 1001 on stent 12 and female interlocking feature 37 on slider 13. Fig. 27 shows stent 12 and slider 13 interlocked.

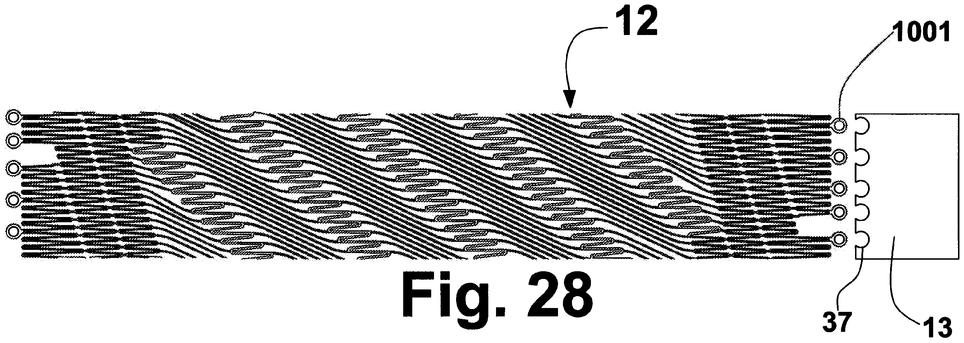

Fig. 28 shows stent 1000 and slider 13 not interlocked. Views of Fig. 27 and Fig. 28 are plan views. The interlocking features of Fig. 27 and Fig. 28 are shown as round; it will be appreciated that the interlocking features can be any geometric shape providing interlocking surfaces. In the preferred embodiment of the present invention, the wall thickness of slider 13 with interlocking features 37, as shown in Fig. 27 and Fig. 28, is thicker than the wall thickness of stent 12 such that slider 13 could readily engage with distal stop 15 during stent reconstraining.

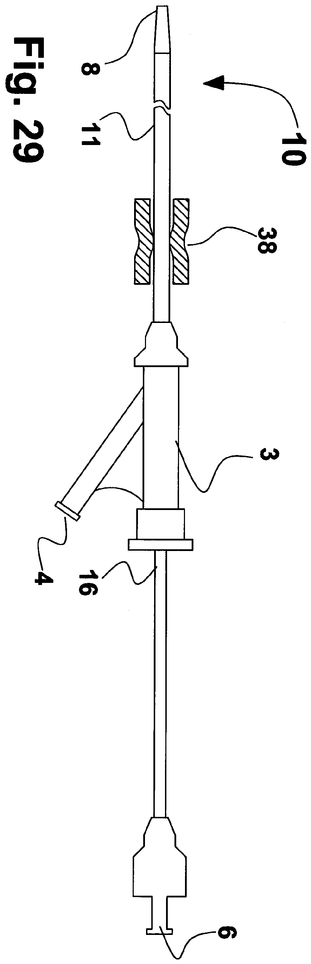

In an alternate embodiment as shown in Fig. 29, gripper 38 (shown in cross-section) is coaxial to outer sheath 11 on stent delivery system 10 near handle 3 such that gripper 38 is always outside the body. During reconstraining, it may be beneficial for the user (physician) to grip outer sheath 11 and pusher 16 (or alternately housing 31, shown in Fig. 25), thereby holding pusher 16 substantially stationary and moving outer sheath 11 distally to reconstrain stent 12 into outer sheath 11. For example, if the length of outer sheath 11 outside the body is such that the tubular portions of stent delivery system 10 would buckle if handle 3 was gripped during the reconstraining, outer sheath 11 could be gripped closer to the access site on the body. Because outer sheath 11 typically has a small diameter and is possibly difficult to grip, gripper 38 which is coaxial with outer sheath 11 can be designed to facilitate the user for gripping outer sheath 11. Gripper 38 can be free to move axially along outer sheath 11, and grip outer sheath 11 when the user applies pressure to gripper 38 or otherwise engages gripper 38 to outer sheath 11. For example, the engagement can be accomplished through a spring mechanism, compliant material selections, or combinations of mechanisms.

The stents may be placed within vessels using procedures well known in the art. The stents may be loaded into the proximal end of a catheter and advanced through the catheter and released at the desired site. Alternatively, the stents may be carried about the distal end of the catheter in a compressed state and released at the desired site. The stents may either be self-expanding or expanded by means such as an inflatable balloon segment of the catheter. After the stent(s) have been deposited at the desired intralumenal site, the catheter is withdrawn.

The stents may be placed within body lumen such as vascular vessels or ducts of any mammal species including humans, without damaging the lumenal wall. For example, the stent can be placed within a lesion or an aneurysm for treating the aneurysm. In one embodiment, the flexible stent is placed in a super femoral artery upon insertion into the vessel. In a method of treating a diseased vessel or duct a catheter is guided to a target site of a diseased vessel or duct. The stent is advanced through the catheter to the target site. For example, the vessel can be a vascular vessel, femoropopliteal artery, tibial artery, carotid artery, iliac artery, renal artery, coronary artery, neurovascular artery or vein.

Stents may be well suited to treating vessels in the human body that are exposed to significant biomechanical forces. Stents that are implanted in vessels in the human body that are exposed to significant biomechanical forces must pass rigorous fatigue tests to be legally marketed for sale. These tests typically simulate loading in a human body for a number of cycles equivalent to 10 years of use. Depending on the simulated loading condition, the number of test cycles may range from 1 to 400 million cycles. For example, stents that are intended to be used in the femorpopliteal arteries may be required to pass a bending test where the stent is bent to a radius of about 20mm 1 to 10 million times or axially compressed about 10% 1 to 10 million times.

It is to be understood that the above-described embodiments are illustrative of only a few of the many possible specific embodiments, which can represent applications of the principles of the invention. For example, a stent could be made with only right-handed or only left-handed helical portions, or the helical strut band could have multiple reversals in winding direction rather than just one. Also, the helical strut band could have any number of turns per unit length or a variable pitch, and the strut bands and/or coil bands could be of unequal length along the stent.

The stent delivery system of the present invention may be used with any stent that allows recapturing after partial deployment.

## Claims

1. A delivery system (10) for a self-expanding stent, said system comprises:
a guide wire tube (14) located coaxially with an outer sheath (11), said guide wire tube (14) and said outer sheath (11) including a distal and proximal end;
a slider (13) located coaxially with said guide wire tube (14) and free to rotate and slide relative to said guide wire tube (14), the slider comprising interlocking features (37) on its distal end that mate and lock with matching interlocking features on the proximal end of a stent (12);
a pusher (16) positioned proximal to said stent (12) and said slider (13);
a distal stop (15) fixed to said guide wire tube (14) distal to said slider (13);
wherein before deployment of the stent, the stent is constrained in a radially compressed state within an inner diameter of said outer sheath (11),
wherein during deployment of the stent said slider can rotate about and move longitudinally along said guide wire tube (14) allowing the stent to move distally or rotate within said outer sheath (11) as said outer sheath is retracted to deploy the stent (12), said pusher (16) in contact with the stent; and
wherein before said outer member (11) is fully retracted, thereby releasing the stent (12), the stent (12) can be re-constrained by moving said guide wire tube (14) and fixed distal stop (15) proximally relative to the stent (12) and said slider (13) until said distal stop (15) contacts said slider (13), then said outer member (11) can be moved distally relative to the stent (12), said slider (13), said guide wire tube (14) and fixed distal stop (15), thereby recapturing the stent inside said outer member (11).

2. The delivery system of claim 1, wherein the interlocking features (37) on said slider (13) are round.

3. The delivery system of claim 1 or claim 2, wherein the interlocking features (37) on said slider (13) are female.

4. The delivery system of any of claims 1 to 3, wherein a wall thickness of the slider (13) is thicker than a wall thickness of the stent (12).

## Patentansprüche

1. Abgabesystem (10) für einen selbstexpandierenden Stent, wobei das System Folgendes beinhaltet:
eine Führungsdrahtröhre (14), welche koaxial mit einer äußeren Hülle (11) angeordnet ist, wobei die Führungsdrahtröhre (14) und die äußere Hülle (11) ein distales und ein proximales Ende beinhalten;
ein Gleitelement (13), welches koaxial mit der Führungsdrahtröhre (14) und frei drehbar und gleitbar in Bezug auf die Führungsdrahtröhre (14) angeordnet ist, wobei das Gleitelement Verriegelungsmerkmale (37) an seinem distalen Ende beinhaltet, die mit passenden Verriegelungsmerkmalen am proximalen Ende eines Stents (12) zusammenpassen und sich damit verriegeln;
einen Schieber (16), welcher proximal zum Stent (12) und zum Gleitelement (13) positioniert ist;
einen distalen Stopper (15), welcher an der Führungsdrahtröhre (14) distal zum Gleitelement (13) befestigt ist;
wobei vor dem Entfalten des Stents, der Stent in einem radial komprimierten Zustand innerhalb eines Innendurchmessers der äußeren Hülle (11) eingezwängt ist,
wobei beim Entfalten des Stents, das Gleitelement um die Führungsdrahtröhre (14) drehen und sich längs der Röhre bewegen kann, wodurch der Stent in die Lage versetzt wird, sich distal zu bewegen oder sich innerhalb der äußeren Hülle (11) zu drehen, während die äußere Hülle zurückgezogen wird, um den Stent (12) zu entfalten, wobei der Schieber (16) mit dem Stent in Kontakt ist; und
wobei, bevor das äußere Glied (11) vollständig eingezogen ist, wodurch der Stent (12) gelöst wird, der Stent (12) wieder eingezwängt werden kann, indem die Führungsdrahtröhre (14) und der feste distale Stopper (15) proximal in Bezug auf den Stent (12) und das Gleitelement (13) bewegt werden, bis der distale Stopper (15) mit dem Gleitelement (13) in Kontakt geht, wonach das äußere Glied (11) distal in Bezug auf den Stent (12), das Gleitelement (13), die Führungsdrahtröhre (14) und den festen distalen Stopper (15) bewegt werden kann, wodurch der Stent innerhalb des äußeren Gliedes (11) wieder eingefangen wird.

2. Abgabesystem nach Anspruch 1, bei welchem die Verriegelungsmerkmale (37) an dem Gleitelement (13) rund sind.

3. Abgabesystem nach Anspruch 1 oder 2, bei welchem die Verriegelungsmerkmale (37) an dem Gleitelement (13) weiblich sind.

4. Abgabesystem nach einem der Ansprüche 1 bis 3, bei welchem eine Wanddicke des Gleitelements (13) dicker ist als die Wanddicke des Stents (12).

## Revendications

1. Système de pose (10) d'un stent auto-expansible, ledit système comprenant :
un tube de fil-guide (14) agencé coaxialement par rapport à une gaine externe (22), ledit tube de fil-guide (14) et ladite gaine externe (11) incluant une extrémité distale et une extrémité proximale ;
un coulisseau (13) agencé coaxialement par rapport audit tube de fil-guide (14) et pouvant tourner et glisser librement par rapport audit tube de fil-guide (14), le coulisseau comprenant des structures à verrouillage mutuel (37) sur son extrémité distale accouplées à des structures à verrouillage mutuel correspondantes et verrouillées sur celles-ci sur l'extrémité proximale d'un stent (12) ;
un poussoir (16) positionné de manière proximale par rapport audit stent (12) et audit coulisseau (13) ;
un arrêt distal (15) fixé sur ledit tube de fil-guide (14), de manière distale par rapport audit coulisseau (13) ;
dans lequel, avant le déploiement du stent, le stent est retenu dans un état à compression radiale dans le cadre d'un diamètre intérieur de ladite gaine externe (11) ;
dans lequel, au cours du déploiement du stent, ledit coulisseau peut tourner autour du tube de fil-guide (14) et se déplacer longitudinalement le long de celui-ci, permettant le déplacement distal ou la rotation du stent dans ladite gaine externe (11) lorsque ladite gaine externe est rétractée pour déployer le stent (12), ledit poussoir (16) étant en contact avec le stent ; et
dans lequel, avant la rétraction complète dudit élément externe (11), libérant ainsi le stent (12), le stent (12) peut être de nouveau contraint en déplaçant ledit tube du fil-guide (14) et l'arrêt distal fixe (15) de manière proximale par rapport au stent (12) et audit coulisseau (13) jusqu'à ce que ledit arrêt distal (15) contacte ledit coulisseau (13), ledit élément distal (11) pouvant alors être déplacé de manière distale par rapport au stent (12), ledit coulisseau (13), ledit tube du fil-guide (14) et ledit arrêt distal fixe (15) capturant de nouveau le stent à l'intérieur dudit élément externe (11).

2. Système de pose selon la revendication 1, dans lequel les structures à verrouillage mutuel (37) sur ledit coulisseau (13) sont rondes.

3. Système de pose selon les revendications 1 ou 2, dans lequel les structures à verrouillage mutuel (37) sur ledit coulisseau (13) sont femelles.

4. Système de pose selon l'une quelconque des revendications 1 à 3, dans lequel une épaisseur de paroi du coulisseau (13) est supérieure à une épaisseur de paroi du stent (12).
